# EUROPEAN PATENT APPLICATION

(11) **EP 1 949 881 A1**
(43) Date of publication of application: **30.07.2008**
(21) Application number: 06810660.8
(22) Date of filing: 27.09.2006
(51) Int. Cl.: A61F 13/56, A61F 5/44, A61F 13/00, A61F 13/15

(54) **ABSORBENT ARTICLE**

(30) Priority: 02.11.2005 JP 2005320047
(71) Applicant: Uni-Charm Corporation, Ehime 799-0111 (JP)
(72) Inventor: UDA, Masashi, Kanonji-shi Kagawa 769-1602 (JP)
(74) Representative: Sperling, Rüdiger
(86) International application number: PCT/JP2006/319202
(87) International publication number: WO 2007/052428

(57) **Abstract**

An absorbent article that having marks indicating an ideal wear position, ensures stable wear condition. There is provided absorbent article (10) having side flaps (12) for fixing to shorts, wherein there are disposed at least either first marks (14) indicating the breadthwise center position of absorbent article main body (11) or second marks (15) indicating of the lengthwise center position of side flap (12) of absorbent article main body (11) so as to ensure stable wear condition.

## Description

### TECHNICAL FIELD

The present invention relates to an absorbent article that is provided with marks indicating ideal attachment positions and thereby ensures stable attachment. In particular, the present invention relates to an absorbent article used along with an undergarment, such as sanitary napkins and urine-absorbing pads.

### BACKGROUND ART

When attaching an absorbent article such as a sanitary napkin, in general, a user attaches it while visually ascertaining the positional relationship between a crotch part of shorts and the sanitary napkin. As used herein, the undergarment to which the sanitary napkin is attached has the crotch part that is narrow and curved to the inside in the vicinity of the crotch. In the sanitary napkin, the rear side, the area corresponding to the crotch part, is shaped so as to increase its width toward the outside in order to cover the hip. The user therefore sets the sanitary napkin, placing the transverse mid-portion of the napkin on the longitudinal centerline of the crotch part. For the sanitary napkin with side flaps, the user further sets the mid-portion of the side flaps in the longitudinal direction of the sanitary napkin at a minimum width portion of the crotch part. The user then folds back and fixes the side flaps against the external surface of the crotch part. Thus, the attachment is completed through the foregoing operations.

Patent Document 1 discloses a sanitary napkin in which, in order to attach the sanitary napkin to an ideal position, a mark is provided at the mid-portion of the sanitary napkin and the mark is directly applied to the private parts, ensuring ideal attachment. Nevertheless, the sanitary napkin disclosed in Patent Document 1 requires that a wearer must pull up the undergarment while supporting the sanitary napkin by herself. This might cause dislocation in the attachment of the sanitary napkin when pulling up the undergarment. Moreover, when attaching the sanitary napkin, the wearer must directly touch at the sanitary napkin, and therefore the wearer's hands might be soiled with menstrual blood and body fluid.

Patent Document 1: Japanese Unexamined Patent Application Publication No. Hei 11-76304

### DISCLOSURE OF THE INVENTION

### Problems to be Solved by the Invention

In the sanitary napkin disclosed in Patent Document 1, neither the transverse mid-portion of the napkin nor the mid-portion of side flaps in the longitudinal direction of the napkin is provided with a distinct one serving as a mark. Since the sanitary napkin has no mark for positioning, the user needs much time for positioning at the time of attachment. It is probable that the attachment position is dislocated back, forth, right, and left. If the attachment position is dislocated, it is impossible to have an absorber sufficiently absorb menstrual blood and body fluid. In some cases, the undergarment and the wearer's skin may be soiled. This may also exert uncomfortable feeling on the wearer during the attachment.

In view of the foregoing problems, it is an object of the present invention to provide an absorbent article that is provided with marks indicating ideal attachment positions, ensuring stable attachment.

### Means for Solving the Problems

The present inventor has had intensive investigation in order to solve the above problems, and has achieved the present invention based on the discovery that the above problems can be solved by an absorbent article having at least one pair of first marks pair indicating a central position in a transverse direction of an absorbent article body, and second marks pair indicating a central position of side flaps in a longitudinal direction of the absorbent article body. More specifically, the present invention provides the following absorbent articles.

According to a first aspect of the present invention, an absorbent article includes an absorbent article body having a longitudinal direction and a transverse direction; and a pair of side flaps that project outward from both side parts in the longitudinal direction of the absorbent article body, respectively, and are folded back and fixed against the external surface of a crotch part of an undergarment at the time of attachment. The absorbent article body has a liquid permeable top sheet disposed on the side of a wearer's skin, a liquid impermeable back sheet disposed on the side of the undergarment, an absorber that is interposed between the top sheet and the back sheet, and that absorbs and retains body fluid. The side flaps have at least one of the top sheet, the back sheet, and side sheets, and has at least one pair of first marks pair indicating a central position in the transverse direction of the absorbent article body, and second marks pair indicating a central position of the side flaps in the longitudinal direction of the absorbent article body.

The absorbent article has the side flaps for fixing the absorbent article body to the undergarment, and has at least one pair of the first marks pair indicating the central position in the transverse direction of the absorbent article body, and the second marks pair indicating the central position of the side flaps in the longitudinal direction of the absorbent article body. The first marks pair is used for positioning with the longitudinal centerline of the crotch part of the undergarment, and the second marks pair is used for positioning with the minimum width portion of the crotch part of the undergarment. These marks enable the user to easily recognize at a glance the positional relationship between the undergarment and the absorbent article when fixing the absorbent article to the undergarment. Hence, in the absorbent article, the attachment position is hardly dislocated back, forth, right, and left, ensuring stable attachment. This enables to suppress dislocations in the attachment position of the absorbent article. It is therefore rare for the menstrual blood and body fluid leaked from the dislocated absorbent article to soil the skin. It is also rare for the dislocated absorbent article to exert uncomfortable feeling on the wearer. Specific examples of the absorbent article are urine-absorbing pads as well as sanitary napkins.

In an absorbent article according to a second aspect of the present invention, the absorbent article body and the side flaps have a seal part formed at a circumferential edge of sheets constituting the absorbent article body and the side flaps, and the first marks pair and the second marks pair are marks formed by the seal part.

This absorbent article is provided with the seal part formed at the circumferential edge of the sheets constituting the absorbent article body and the side flaps. The first marks pair and the second marks pair can be formed by imparting a characteristic shape to the seal part formed at the circumferential edge of the sheet. That is, the seal used for connecting the sheets is employed to form the first marks pair and the second marks pair. It is therefore unnecessary to install a new manufacturing unit and use a new special material in the manufacturing steps. Hence, the additional cost occasioned by the incorporation of the marks is avoidable.

In an absorbent article according to a third aspect of the present invention, the side flaps have, on the side of the back sheet, an adhesion part that fixes the side flaps folded back against the external surface of the crotch part of the undergarment at the time of attachment, and the adhesion part is disposed so as not to overlap with the seal part.

In this absorbent article, the adhesion part, which fixes the side flaps folded back against the external surface of the crotch part of the undergarment at the time of attachment, is provided on the side of the back sheet of the side flaps. Usually, the adhesion part can be formed by the application of hot melt adhesive (HMA). The seal part can be formed with a connecting device provided with a pattern roll having a pressing part with projections, and a pattern roll with a smooth surface. Consequently, if the adhesion part is overlapped with the seal part, the HMA may be separated from the side flaps when stripping the side flaps from the undergarment, and then the HMA may remain in the undergarment. However, the absorbent article in accordance with the third aspect is capable of avoiding this drawback. In cases where the user attaches an absorbent article with side flaps, the user usually pinches the mid-portion of the side flaps by the fingers, and with the side flaps spread, folds back them against the external surface of the crotch part of the undergarment, and then presses the side flaps by the fingers. Therefore, if the adhesive part is continuously provided in the longitudinal direction of the absorbent article body, when pinching the mid-portion of the flaps, the HMA may stick to the fingers, and the adhesion may be lowered. However, the absorbent article in accordance with the third aspect is also capable of solving this drawback.

In an absorbent article according to a fourth aspect of the present invention, at least one pair of the first marks pair and the second marks pair are marks formed by printing.

In this absorbent article, at least one of the first marks pair and the second marks pair are printed. No particular limitations are imposed on the printing method and the mark shape. This absorbent article is capable of producing with a relatively simple technique the same effect as the absorbent article in accordance with the first aspect.

In an absorbent article according to a fifth aspect of the present invention, at least one pair of the first marks pair and the second marks pair are formed with adhesive paper.

In this absorbent article, at least one pair of the first marks pair and the second marks pair are formed by adhesive paper. The adhesive paper itself can be used as a mark. Alternatively, a marked paper may be used as a mark. Sticking the paper that can serve as a mark achieves the same effects as the absorbent article in accordance with the first aspect.

In an absorbent article according to a sixth aspect of the present invention, there are further provided a pair of solid gathers that are disposed on both sides in a longitudinal direction of the top sheet so as to extend along the longitudinal direction, and stand on the side of a wearer's skin during attachment. Each of the solid gathers has a resilient member extending in the longitudinal direction, and a liquid impermeable gather sheet that is connected integrally with the resilient member, one side end of the gather sheet being a free end. The second marks are marks constructed of a side seal part formed by connecting the other side end of the gather sheet to the top sheet.

This absorbent article has the pair of solid gathers that are disposed on both sides in the longitudinal direction of the top sheet so as to extend along the longitudinal direction, and stand on the side of the wearer's skin during attachment. A characteristic shape is imparted to the side seal part that connects the solid gather and the top sheet, and the characteristic shape can serve as the second marks. Usually, when attaching an absorbent article with side flaps, the side flaps are folded back and fixed against the external surface of the crotch part of the undergarment. After they are fixed, the user cannot visually observe the side flaps. Therefore, even if any mark is provided on the side flaps, it is difficult for the user to ascertain any dislocation in the attachment position after the operation of sticking the side flaps to the undergarment is completed. On the other hand, in the absorbent article in accordance with the sixth aspect, the absorbent article body has the second marks, instead of providing marks on the side flaps. This enables the user to ascertain whether the attachment position is dislocated or not by the aid of the second marks even after the operation of sticking the side flaps to the undergarment.

### Effects of the Invention

Thus, the present invention can provide the absorbent article that has the marks indicating an ideal attachment position and thereby ensures stable attachment.

### BRIEF DESCRIPTION OF THE DRAWINGS

Fig. 1 is a plan view of an absorbent article according to a first preferred embodiment of the present invention;
Fig. 2 is an enlarged view of first marks pair in Fig. 1;
Fig. 3 is a plan view of an absorbent article according to a second preferred embodiment;
Fig. 4 is an enlarged view of first marks pair in Fig. 3;
Fig. 5 is a plan view of an absorbent article according to a third preferred embodiment;
Fig. 6 is a plan view of an absorbent article according to a fourth preferred embodiment;
Fig. 7 is a plan view of an absorbent article according to a modification 1 of the fourth preferred embodiment;
Fig. 8 is a plan view an absorbent article according to a modification 2 of the fourth preferred embodiment;
Fig. 9 is a plan view of an absorbent article according to a fifth preferred embodiment;
Fig. 10 is a plan view of an absorbent article according to a sixth preferred embodiment; and
Fig. 11 is a plan view of an absorbent article according to a seventh preferred embodiment.

### PREFERRED MODE FOR CARRYING OUT THE INVENTION

Preferred embodiments of the present invention will be described below with reference to the accompanying drawings. In the preferred embodiments except for a first preferred embodiment, the construction and the operative effects identical to those of the first preferred embodiment will be left out in the following description.

### First Preferred Embodiment

### [Overall Construction]

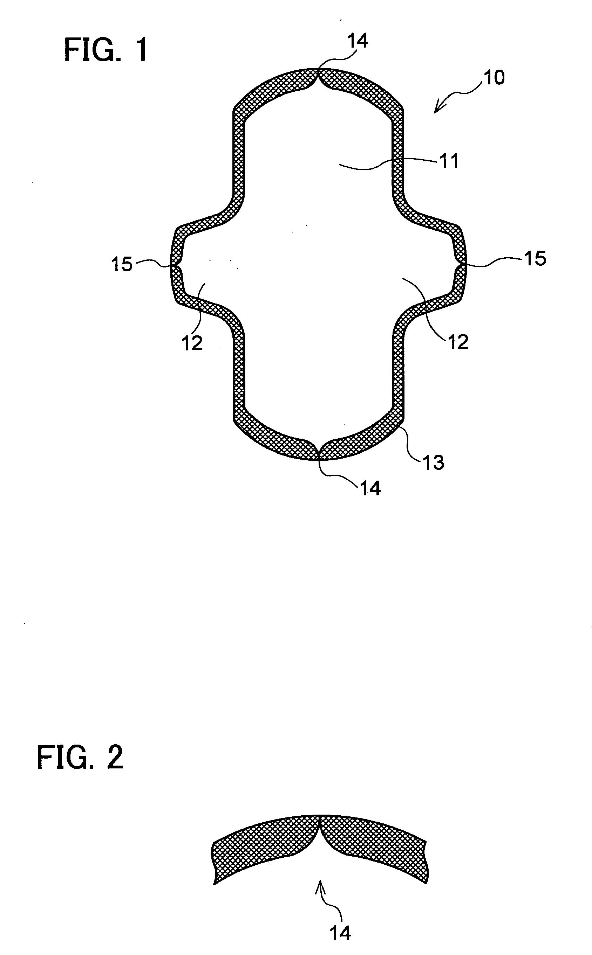

Figure 1 is a plan view when an absorbent article 10 according to the first preferred embodiment is viewed from the side of a wearer's skin, namely from the side of a top sheet. As shown in Fig. 1, the absorbent article 10 has an absorbent article body 11 having a longitudinal direction and a transverse direction, and a pair of side flaps 12. The pair of side flaps 12 project from both side parts in the longitudinal direction of the absorbent article body 11, respectively, and are folded back and fixed against the external surface of a crotch part of the undergarment at the time of attachment. A seal part 13 is provided at the circumferences of the absorbent article body 11 and the side flaps 12. The seal part 13 in the transverse direction of the absorbent article body 11 has a pair of first marks 14, and the seal part 13 in the longitudinal direction of the absorbent article body 11 has a pair of second marks 15.

### [Absorbent Article Body 11]

The absorbent article body 11 has a liquid permeable top sheet that is disposed on the side of a wearer's skin, a liquid impermeable back sheet that is disposed on the side of the undergarment, and an absorber that is interposed between the top sheet and the back sheet, and that absorbs and retains body fluid. Alternatively, the absorbent article body 11 may have a liquid impermeable leakage-proof sheet between the absorber and the back sheet. In this case, the top sheet and the back sheet are connected to each other, and the leakage-proof sheet is also connected to the top sheet and the back sheet.

### Top Sheet

As a sheet member constituting the top sheet, in general, a liquid hydrophilic member having liquid permeability can be used. Preferably, the sheet member is a member of rich flexibility and superior feeling because it is brought into contact with the user's skin. For example, there are liquid permeable nonwoven fabric constructed of polypropylene and polyethylene, artificial fibers such as film, porous plastic film and porous foam, and natural fibers such as cotton. As the sheet member, the exemplified members may be used singularly or in combination of these.

### Back Sheet

As a sheet member constituting the back sheet, there can be used a liquid impermeable resin film and a nonwoven fabric that are constructed of one of or in combination of polypropylene, polyethylene, polyethylene terephthalate, and ethylene vinyl acetate. Preferably, the sheet member constituting the back sheet is a nonwoven fabric with resin films laminated thereon, a laminate member of resin and paper, and a member to which liquid permeability is imparted by adding filler or the like to a resin film, followed by drawing. Absorber

Although no particular limitation is imposed on the absorber, for example, such a member that pulverized pulp (e.g., wood pulp) is covered with a wrap member (tissue) may be used. Examples of the sheet member are air laid pulp, span lace nonwoven fabric and melt blown nonwoven fabric each using hydrophilic fiber, water absorptive paper, and members in combination of these, as well as starch polymer, acrylic acid polymer, and celluloses. No particular limitation is imposed on the shape of the material of the absorber. For example, the absorber may have the shape of granule, sheet, or fiber. No particular limitation is also imposed on the shape of the absorber itself.

### [Side Flaps 12]

The side flaps 12 have at least one of the top sheet, back sheet, and the side sheets. The sheet member constituting the side sheets may be a liquid permeable sheet or a liquid impermeable sheet. The side sheets may be arranged so as to project outward from between the top sheet and the back sheet, respectively, in the both side parts in the longitudinal direction of the absorbent article body 11, or may be arranged so as to project outward from the upper part of the top sheet, respectively. Alternatively, the side sheets may be arranged so as to project outward from the lower part of the back sheet. In a still other alternative, the top sheet itself constituting the absorbent article body 11 may be arranged so as to project outward from the both side parts in the longitudinal direction, respectively.

In the first preferred embodiment, the second marks indicate the central positions of the side flaps 12 in the longitudinal direction of the absorbent article body 11, and are disposed at the seal part 13 in the circumferences of the right and left side flaps 12. Usually, in the side flaps 12, adhesion parts are disposed on the side of the back sheet (not shown). The adhesion parts are provided for fixing the side flaps 12 folded back against the external surface of the crotch part of the undergarment at the time of attachment (not shown).

### [Seal Part 13]

A connecting device provided with a pattern roll having a pressing part with projections and a pattern roll with a smooth surface is used to form the seal part 13. The connecting device forms the seal part 13 by connecting the circumferences of the top sheet and that of the back sheet with each other. In order to improve the touch of the seal part 13, it is usually preferable that the connection is carried out by pressing the pattern roll having the pressing part with projections from the side of the top sheet, and pressing the pattern roll with the smooth surface from the side of the back sheet. The same connecting device is used to seal the leakage-proof sheet, the top sheet, and the back sheet.

### [Pair of First Marks 14 and Pair of Second Marks 15]

The pair of first marks 14 are marks indicating the central positions in the transverse direction of the absorbent article body 11. The pair of second marks 15 are marks indicating the central positions of the side flaps 12 in the longitudinal direction of the absorbent article body 11. As shown in Fig. 1, the pair of first marks 14 and the pair of second marks 15 are of the same shape, and the shape of arbitrary marks can be obtained by slitting the seal part 13. These marks are not necessarily required to have the same shape as in this preferred embodiment, and they may have different shapes. Figure 2 is an enlarged view of the pair of first marks 14. As shown in Fig. 2, the pair of first marks 14 are marks obtained by forming a deep slit that reaches the end, and the pair of second marks 15 have the same shape (not shown). Both of the pair of first marks 14 and the pair of second marks 15 can be formed by imparting a slit shape to the pattern rolls used for forming the seal part 13. Specifically, to form the pair of first marks 14 and the pair of second marks 15, slits are provided respectively at the mid-portion of both edges in the longitudinal direction of the absorbent article body 11, and at the portions corresponding to the mid-portions of the side edges of the side flaps 12 in the longitudinal direction of the absorbent article body 11, on the side of the pattern roll having the pressing part with projections. Then, sealing is performed with the connecting device, thereby forming the pair of first marks 14 and the pair of second marks 15.

### [Operative Effects]

The pair of first marks 14 indicates the central positions in the transverse direction of the absorbent article body 11, and used to perform positioning with the centerline in the longitudinal direction of the crotch part of the undergarment. The pair of second marks 15 indicates the central positions of the side flaps 12 in the longitudinal direction of the absorbent article body 11, and used to perform positioning with the minimum width part of the crotch part of the undergarment. These marks enable the user to easily recognize at a glance the positional relationship between the undergarment and the absorbent article 10 when fixing the absorbent article 10 to the undergarment. Thus, the attachment position of the absorbent article 10 is hardly dislocated back, forth, right, and left, enabling the user to ensure stable attachment.

### Second Preferred Embodiment

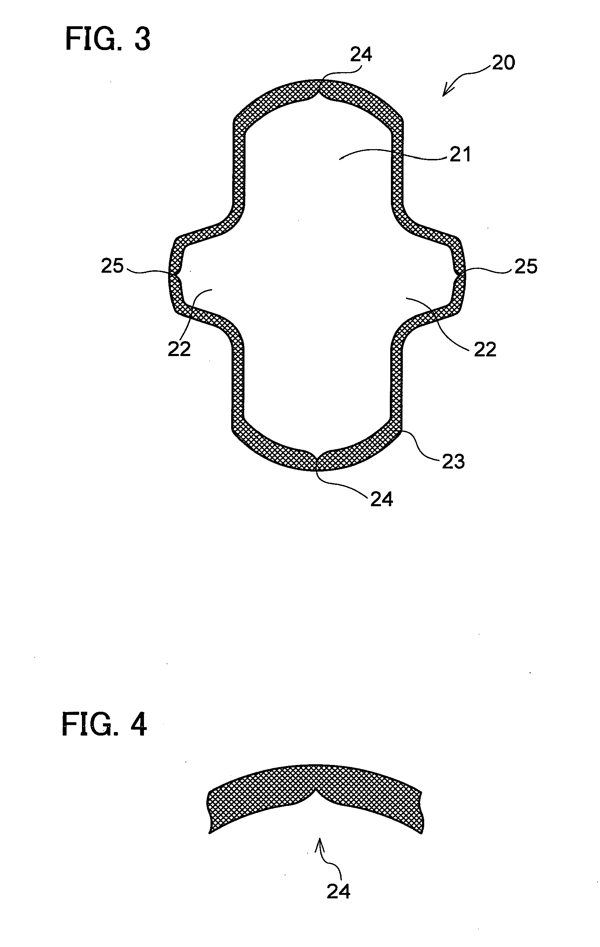

Figure 3 is a plan view when an absorbent article 20 according to a second preferred embodiment is viewed from the side of a wearer's skin, that is, from the side of the top sheet. As shown in Fig. 3, the absorbent article 20 is different from the absorbent article 10 of the first preferred embodiment in the shape of the pair of first marks 14 and the pair of second marks 15. Otherwise, the construction is identical to that described in the first preferred embodiment. Specifically, the pair of first marks 14 and the pair of second marks 15 of the first preferred embodiment are shaped like a deep slit extending to the end, whereas those of the second preferred embodiment are shaped like a shallow slit, instead of the deep slit extending to the end. Figure 4 is an enlarged view of first marks 24. Referring to Fig. 4, unlike the first preferred embodiment, in the absorbent article 20 of the second preferred embodiment, any unsealed portion is not present at the ends of the respective marks, and the ends of the marks are firmly sealed. This avoids the fear that the sheets are stripped from each other from an unsealed point. Third Preferred Embodiment

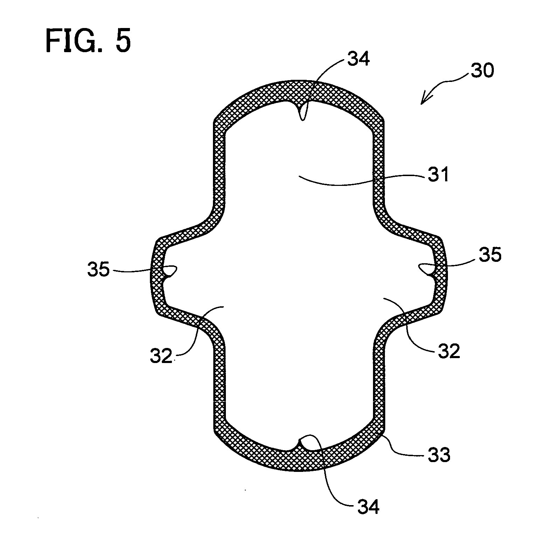

Figure 5 is a plan view when an absorbent article 30 according to a third preferred embodiment is viewed from the side of a wearer's skin, that is, from the side of the top sheet. As shown in Fig. 5, like the second preferred embodiment, the absorbent article 30 of the third preferred embodiment is different from the absorbent article 10 of the first preferred embodiment in the shape of the pair of first marks 14 and the pair of second marks 15. Otherwise, the construction is identical to that described in the first preferred embodiment. Specifically, first marks 34 and second marks 35 of the absorbent article 30 of the third preferred embodiment are shaped so as to project at an acute angle inwardly of an absorbent article body 31. Compared to the first and second preferred embodiments in which the slits are provided in the seal part as marks, the seal strength can be further enhanced, enabling to further avoid the fear of stripping between the sheets.

### Fourth Preferred Embodiment

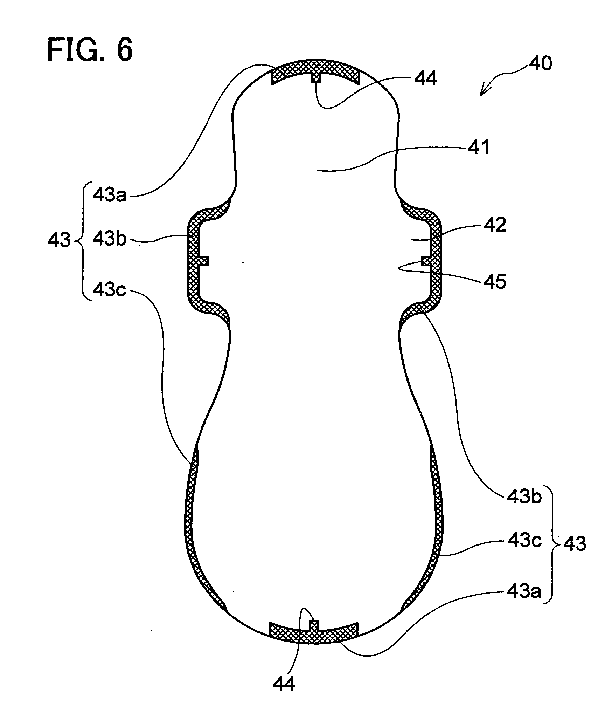

Figure 6 is a plan view when an absorbent article 40 according to a fourth preferred embodiment is viewed from the side of a wearer's skin, that is, from the side of the top sheet. As shown in Fig. 6, the absorbent article 40 is different from the first preferred embodiment in the shape of the absorbent article body, the region where the seal part is disposed, and the shape of the marks. Otherwise, the construction is identical to that described in the first preferred embodiment. Specifically, the locations of side flaps 42 are shifted to the front side of an absorbent article body 41 (the term "the front side" as used herein means the side to be set at the front of the undergarment during attachment). Both side parts in the longitudinal direction of the rear side of the absorbent article body 41 (the term "the rear side" as used herein means the side to be set at the rear of the undergarment during attachment) are swelled slightly outward, resulting in a wider shape than the front side. A seal part 43 is not disposed throughout the circumference of the absorbent article 40. That is, the seal part 43 is disposed at the circumference at both ends in the longitudinal direction of the absorbent article body 41, at part of both side parts in the longitudinal direction on the rear side, and at the circumferences of the side flaps 42. First marks pair 44 and second marks pair 45 are formed by allowing the seal part 43 to project in the shape of a band inwardly of the absorbent article body 41. Thus, in the absorbent article 40 of the fourth preferred embodiment, the seal part 43 is disposed at a minimum required area to a degree that the stripping between the sheets does not occur. The fourth preferred embodiment produces the same effects as the first

### preferred embodiment.

### Modification 1

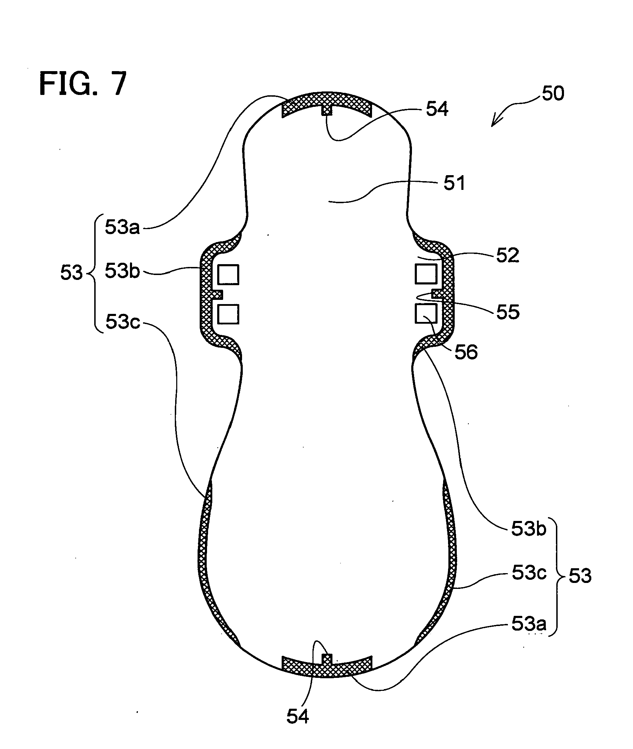

Figure 7 is a plan view when an absorbent article 50 according to a modification 1 of the fourth preferred embodiment is viewed from the side of a wearer's skin, that is, from the side of the top sheet. As shown in Fig. 7, in the absorbent article 50 of this modification, the above-mentioned side flaps 42 of the absorbent article 40 are provided with adhesion parts 56 for fixing the side flaps 42 folded back against the external surface of the crotch part of the undergarment at the time of attachment. Specifically, a plurality of the adhesion parts 56 are located at a predetermined spacing so as not to overlap with a seal part 53. Usually, hot melt adhesive (HMA) is applied as the adhesion parts 56.

This modification produces the following operative effects. That is, a seal part usually has irregularities, and therefore if the adhesive part is overlapped with the seal part, the HMA may be separated from the side flaps when stripping the side flaps from the undergarment. There may arise the drawback that the separated HMA remains in the undergarment. However, this drawback can be solved by this modification. Especially, like this modification, when the seal part 53 projects to the inside of the absorbent article body 51, as the mark of the corresponding locations, it is effective to provide the plurality of adhesive parts at a predetermined spacing. Additionally, when attaching an absorbent article with side flaps, the user usually pinches the mid-portion of the side flaps by the fingers, and with the side flaps spread, folds back them against the external surface of the crotch part of the undergarment, and then presses the side flaps by the fingers. Therefore, if the adhesive part is continuously provided in the longitudinal direction of the absorbent article body, when pinching the mid-portion of the flaps, the HMA may stick to the fingers, and the adhesion may be lowered. However, this modification is also capable of solving this drawback.

### Modification 2

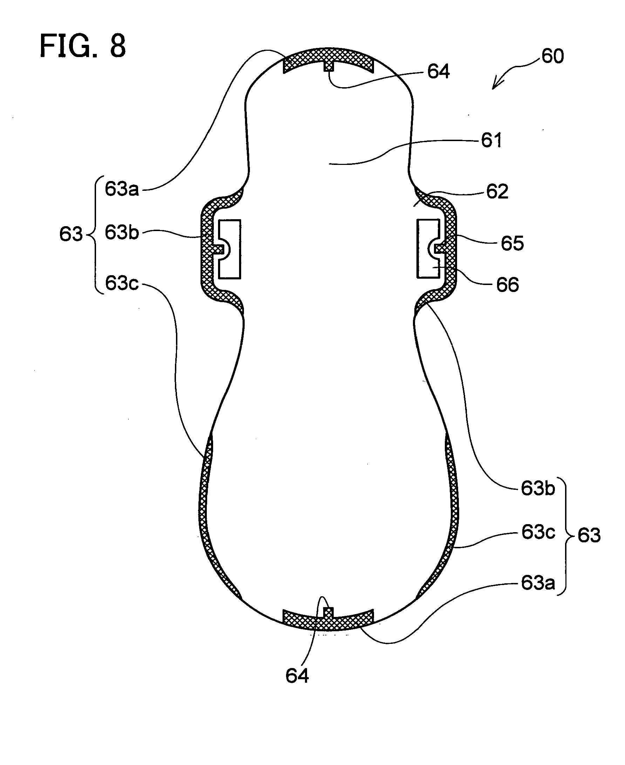

Figure 8 is a plan view when an absorbent article 60 according to a modification 2 of the fourth preferred embodiment is viewed from the side of a wearer's skin, that is, from the side of the top sheet. As shown in Fig. 8, adhesive parts 66 are provided continuously so as to detour second marks 65, whereas in the modification 1, the plurality of adhesion parts 56 of the absorbent article 50 are located at the spacing. Because the adhesive part 66 is provided continuously, the modification 2 exhibits the enhanced adhesive and also produces the same effects as the modification 1.

### Fifth Preferred Embodiment

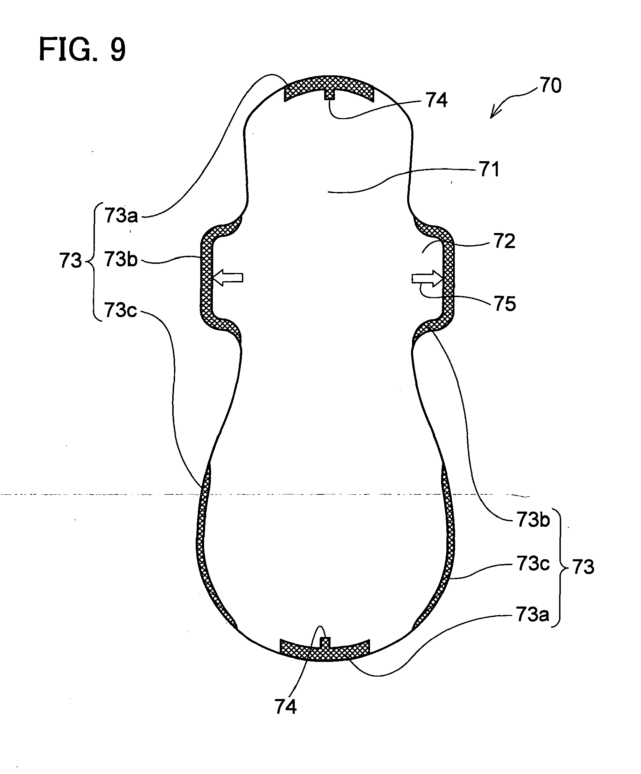

Figure 9 is a plan view when an absorbent article 70 according to a fifth preferred embodiment is viewed from the side of a wearer's skin, that is, from the side of the top sheet. As shown in Fig. 9, the absorbent article 70 has printed second marks pair 75, instead of the pair of second marks 45 of the absorbent article 40 in the fourth preferred embodiment. In the fifth preferred embodiment, pair first marks 74 are formed by allowing a seal part 73 to project to the inside of an absorbent article body 71. Alternatively, the pair of first marks 74 may also be printed like the pair of second marks 75. In the fifth preferred embodiment, the pair of second marks 75 are in the shape of an arrow. No particular limitation is imposed on the shape of the marks so long as they can serve as the marks. No particular limitation is imposed on the printing method. Examples of the printing method are conventionally well-known ink printing and braille printing using emboss. The printed second marks are required to be visually observable from the side sheet side. In order to avoid sticking to the skin, it is therefore preferable that, in place of the top side of the side sheets (the wearer's skin side), the back side of the side sheets (the undergarment side) or the top side of the back sheet is subjected to printing, enabling the printed marks to be visible from the side sheets side. The absorbent article 70 so constructed in accordance with the fifth preferred embodiment is also capable of producing the same effects as the first preferred embodiment.

### Sixth Preferred Embodiment

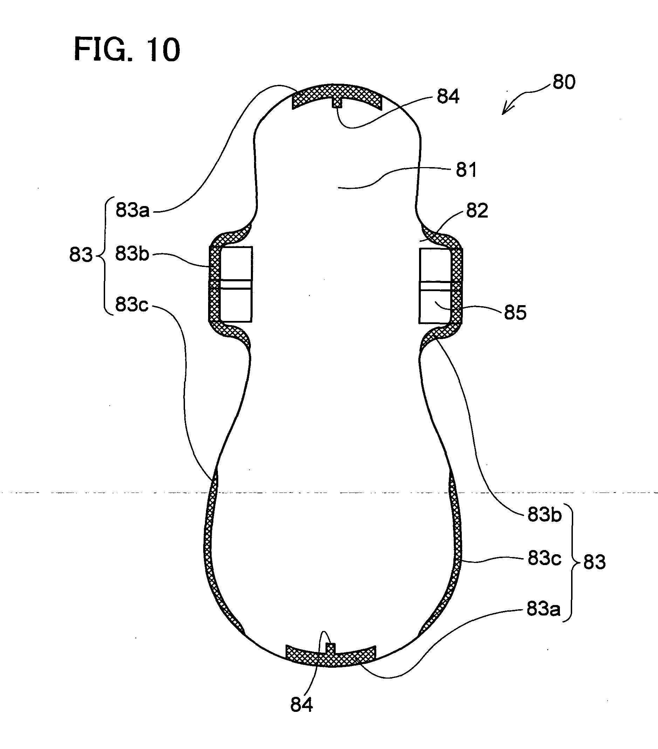

Figure 10 is a plan view when an absorbent article 80 according to a sixth preferred embodiment is viewed from the side of a wearer's skin, that is, from the side of the top sheet. As shown in Fig. 10, the absorbent article 80 has second marks pair 85 that can be formed by sticking papers each provided with a mark, to the side sheets, in place of the pair of second marks45 of the absorbent article 40 in the fourth preferred embodiment. Specifically, the pair of second marks 85 are required to be visually observable from the side sheets side. In order to avoid sticking to the skin, it is therefore preferable that, instead of direct sticking to the side sheets side (the wearer's skin side), sealing is carried out after inserting papers each having a mark into between the side sheets and the back sheet, enabling the marks to be visually observable from the side sheets side. No particular limitation is imposed on the shape of the marks. The absorbent article 80 so constructed in accordance with the sixth preferred embodiment is also capable of producing the same effects as the first preferred embodiment.

### Seventh Preferred Embodiment

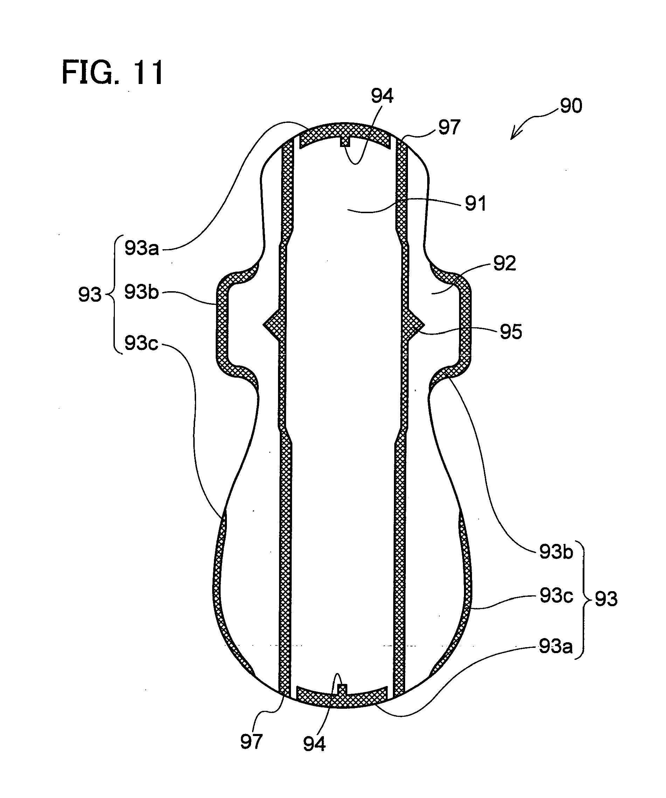

Figure 11 is a plan view when an absorbent article 90 according to a seventh preferred embodiment is viewed from the side of a wearer's skin, that is, from the side of the top sheet. As shown in Fig. 11, the absorbent article 90 has a pair of solid gathers disposed so as to extend along the longitudinal direction on both sides in the longitudinal direction of the top sheet (for the sake of simplicity, the solid gathers are not illustrated). Second marks pair 95 can be formed by side seal parts 97 that connect the solid gathers and the top sheet to each other.

The solid gathers stand on the wearer's skin side during attachment in order to prevent leakage. Each of the solid gathers has a resilient member (not shown) disposed along the longitudinal direction of an absorbent article body 91, and a liquid impermeable gather sheet (not shown) that is integrally connected to the resilient member, one side end of the gather sheet being a free end. As the resilient member and the gather sheet, conventionally well-known members can be used. The pair of second marks 95 can be formed by the side seal parts 97 that can be formed by connecting the other side end of the gather sheet to the top sheet. Like a seal part 93 disposed at the circumference of the absorbent article 90, the side seal parts 97 can be formed by using a pattern roll with a smooth surface and a pattern roll having a pressing part with projections. Although in the seventh preferred embodiment, the pair of second marks 95 are shaped so as to project outward at an acute angle, no particular limitation is imposed on the shape of the pair second marks 95 so long as they can serve as the marks. In the absorbent article provided with the solid gathers as in the seventh preferred embodiment, overlapping of the seal part with the ends of the solid gathers may cut the resilient member or inhibit the standing of the solid gathers. Hence, it is preferable that the seal part is not provided throughout the circumference of the absorbent article, namely, the seal part is provided in the area except for the positions overlapped with the solid gathers.

In addition to the same effects as the first preferred embodiment, the absorbent article 90 of the seventh preferred embodiment produces the following effects. In a state in which the absorbent article with side flaps is attached, the side flaps are usually folded back and fixed against the external surface of the crotch part of the undergarment, making it impossible for the user to visually recognize the side flaps. Therefore, even if any mark is provided on the side flaps, it is difficult for the user to ascertain any dislocation in the attachment position after the operation of sticking the side flaps to the undergarment is completed. On the other hand, in the absorbent article of the seventh preferred embodiment, the pair of second marks 95 are provided on the absorbent article body 91, not on the side flaps 92. This enables the user to ascertain whether the attachment position is dislocated or not even after the operation of sticking to the undergarment.

## Claims

1. An absorbent article comprising:
an absorbent article body having a longitudinal direction and a transverse direction, and having a liquid permeable top sheet, a liquid impermeable back sheet, and an absorber that is interposed between the top sheet and the back sheet, and absorbs and retains body fluid;
a pair of side flaps that are constructed of at least one of the top sheet, the back sheet, and side sheets, and that extend outward from both side parts in the longitudinal direction of the absorbent article body, respectively, and are folded back and fixed against an external surface of a crotch part of an undergarment at the time of attachment; and
at least one pair of first marks pair indicating a central position in the transverse direction of the absorbent article body, and second marks pair indicating a central position of the side flaps in the longitudinal direction of the absorbent article body.

2. The absorbent article according to claim 1 wherein,
the absorbent article body and the side flaps have a seal part formed at a circumferential edge of sheets constituting the absorbent article body and the side flaps; and
the first marks and the second marks are marks formed by the seal part.

3. The absorbent article according to claim 2 wherein the first marks are a pair of marks formed so as to face each other in the longitudinal direction of the absorbent article body.

4. The absorbent article according to claim 2 wherein the second marks are a pair of marks formed so as to face each other in the transverse direction of the absorbent article body.

5. The absorbent article according to claim 1 wherein,
the side flaps have, on the side of the back sheet, an adhesion part for fixing the side flaps folded back against the external surface of the crotch part of the undergarment at the time of attachment; and
the adhesion part is disposed so as not to overlap with the seal part.

6. The absorbent article according to claim 1 wherein at least one of the first marks and the second marks are marks formed by printing.

7. The absorbent article according to claim 1 wherein at least one of the first marks and the second marks are formed by adhesive paper.

8. The absorbent article according to claim 1, further comprising:
a pair of solid gathers that are disposed on both sides in a longitudinal direction of the top sheet so as to extend along the longitudinal direction, and stand on the side of a wearer's skin during attachment, wherein,
each of the solid gathers has a resilient member extending in the longitudinal direction, and a liquid impermeable gather sheet connected integrally with the resilient member, one side end of the gather sheet being a free end; and
the second marks are marks formed by side seal parts formed by connecting the other side end of the gather sheet to the top sheet.
